# EUROPEAN PATENT SPECIFICATION

(11) **EP 2 694 128 B1**
(45) Date of publication and mention of the grant of the patent: **07.12.2016**
(21) Application number: 12716297.2
(22) Date of filing: 04.04.2012
(51) Int. Cl.: A61M 1/36

(54) **TEMPERATURE CONTROL DEVICE FOR USE IN FLUID-BASED HYPER/HYPOTHERMIA SYSTEMS**
TEMPERATURREGELUNGSVORRICHTUNG ZUR VERWENDUNG IN HYPER-/HYPOTHERMIESYSTEMEN AUF FLUIDBASIS
DISPOSITIF DE RÉGULATION DE LA TEMPÉRATURE DESTINÉ À ÊTRE UTILISÉ DANS DES SYSTÈMES D'HYPER/HYPOTHERMIE À BASE DE FLUIDE

(30) Priority: 08.04.2011 DE 102011016508
(43) Date of publication of application: 12.02.2014
(60) Divisional application: 16195495.3
(73) Proprietor: Sorin Group Deutschland GmbH, 80939 München (DE)
(72) Inventor: KNOTT, Erwin, 85586 Poing (DE); FRONHÖFER, Manfred, 81379 München (DE)
(74) Representative: Hoffmann Eitle
(86) International application number: PCT/EP2012/056154
(87) International publication number: WO 2012/136700

(56) References cited:
- EP-A1- 1 970 080
- WO-A1-2006/063080
- US-A- 6 156 007
- US-A1- 2007 020 142
- US-A1- 2010 143 192
- US-B2- 6 581 403

## Description

The invention relates to a temperature control device for use in fluid-based hyper/hypothermia systems.

A fluid-based hyper/hypothermia system is disclosed, for example, in DE 696 34 572 T2.

US 6,581,403 B2 teaches a heating/cooling system for indwelling heat exchange catheter that includes a heat exchange bath that is configured to receive a conduit that carries saline to and from the catheter. A heating/cooling fluid is in the bath and exchanges heat with the saline. The heating/cooling fluid flows through a heat exchanger that includes a refrigerant and a variable speed DC compressor for removing heat from the refrigerant. A coolant pump circulates the heating/cooling fluid between the heat exchanger and the heat exchange bath.

Fluid-based hyper/hypothermia systems which use a temperature-controlled fluid to raise the temperature of a human or animal body, body part or organ to above the normal core body temperature or to lower it to below the normal core body temperature require a temperature control device that provides a temperature-controlled fluid, by means of which the desired change in body temperature can be effected. The temperature of the fluid must be controlled in the temperature control device in accordance with the quantity of heat to be supplied to or removed from the body, i.e. it must be heated or cooled and then maintained at a predetermined temperature.

In order to heat or cool the fluid in the temperature control device, energy is required that is provided as a general rule by the local power network. Thus, a conventional temperature control device comprises a power supply which allows the temperature control device to be connected to the local power network. Both the power supply as well as numerous individual components of the temperature control device must be adapted to the local power network. Since there are different local power networks in different regions of the world, the region of the world in which the temperature control device is ultimately supposed to be used and the specifications of the local power network according to which the power supply of the temperature control device and the temperature control device itself have to be configured must, with a considerable amount of effort, always be taken into consideration when constructing a temperature control device for hyper/hypothermia applications.

The aim of the invention is to simplify this aspect of the construction of a temperature control device and to provide a temperature control device for hyper/hypothermia systems that can be used in different regions of the world owing to a simple adaptation.

This aim is achieved by a temperature control device having the features specified in patent claim 1. Advantageous embodiments are revealed by the sub-claims.

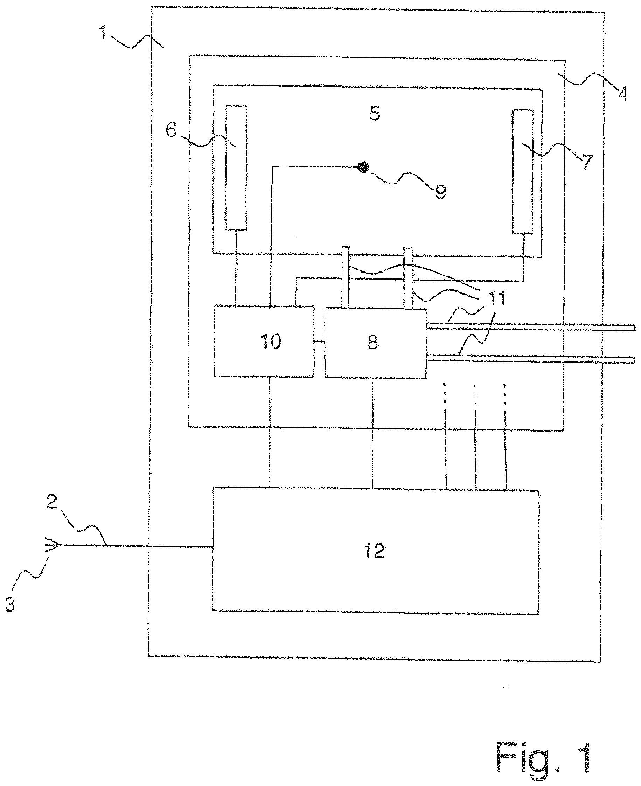

The invention is explained in the following by means of an embodiment and with reference to Fig. 1.
- Fig. 1: shows an embodiment of a temperature control device according to the invention;
- Fig. 2: shows a further embodiment of the temperature control device according to the invention.

The embodiment of a temperature control device 1 according to the invention, which is to be used in fluid-based hyper/ hypothermia systems and is shown in Fig. 1, comprises a connection unit 2 for connecting the device to a local power network 3. In Germany, this is the general AC network of 220/380 V at 50 Hz, in Japan it is an AC network of 100 V at, for example, 60 Hz, and in the USA it is, in turn, an AC network of 120 V at 60 Hz. These differences, in particular also in the frequencies of the local power networks, lead to differences in the leakage currents which result from the change of the connected alternating current over time and with regard to which strict guidelines apply, in particular for medical-technical systems in a surgical environment since the effect of electrical currents in the case, for example, of open heart surgeries must remain minimal. In order to minimise these leakage currents, the electric lines in conventional temperature control devices must have certain insulations. This leads to increased material costs since in particular the insulation can age and must then be replaced if the guidelines with respect to the leakage currents are no longer met.

The temperature control device according to the invention is connected to the power network 3 via the connection unit 2 and can draw the power required to control the temperature of the fluid from the power network.

The temperature control of the fluid is carried out by means of a fluid temperature control unit 4 which comprises the components that are required for heating or cooling the fluid. These normally include a fluid container 5, a heater 6, a cooler 7, a supply pump 8, a temperature sensor 9 and a temperature controller 10, which are shown in Fig. 1 merely in schematic form and as an example of the components of the fluid temperature control unit 4. In the present embodiment, the supply pump 8 works, for example, with a direct current motor whereas the cooler 7 has a direct current compressor. Also shown by way of an example are pipelines 11, via which the pump 8 removes the fluid from the fluid container 5 and conveys it to the outside such that it can be used in the hyper/hypothermia system, or via which the fluid is conveyed out of the hyper/hypothermia system back into the fluid container 5. The pump can also be provided in the hyper/ hypothermia system such that it can be omitted from the fluid temperature control unit 4 of a temperature control device 1 as according to the invention. Depending on the hyper/ hypothermia system in which the temperature control device 1 is used, other components, such as a stirrer for the fluid in the fluid container 5, may be added to/omitted from the fluid temperature control unit 4.

In order to supply power to the electrical consumers, i.e., for example, the heater 6, the cooler 7, the supply pump 8 and the temperature controller 10, of the fluid temperature control unit of a temperature control device 1 according to the invention, a power supply unit 12 is provided according to the invention, via which all of the electrical consumers of the fluid temperature control unit 4 are electrically supplied with constant connected loads, i.e. irrespective of the local power network. According to the invention, direct current is supplied, for example with a supply voltage of 48 V and a power of up to 3.5 kW. This means that according to the invention, the consumers of the fluid temperature control unit 4 which are supplied via the power supply unit are all not directly connected to the power network 3 and do not have to be designed for this power network, but are rather all supplied with direct current by the power supply unit 12 according to the invention. Different consumers can thereby be supplied with different voltages/powers which are provided by the power supply unit 12 according to the invention, and this is indicated in Fig. 1 by the connections between the power supply unit 12 and the fluid temperature control unit 4 which are dashed at one end. The power supply unit 12 according to the invention thereby performs adaptation to the local power network and conversion to a power supply with constant connected loads.

By means of the power supply unit according to the invention, it is achieved that the temperature control device can be adapted to the conditions of a regional power network with an acceptable amount of effort. The adaptation to the local power network of the region in which the device is to be used is achieved by an appropriate design of the power supply unit, which, on the side facing the connection unit, must be designed for connection to the local power network, whereas on the side facing the fluid temperature control unit, the aforementioned uniform power supply with direct current is ensured irrespective of the local power network.

Suitable as the power supply unit according to the invention are standard power supplies (also switched-mode power supplies) that provide, as standard, one or more supply voltages which are required by the fluid temperature control unit so that the temperature of the fluid can be controlled.

It is achieved with the power supply unit as provided according to the invention that the fluid temperature control unit is electrically separate from the local power network. As a result, an improved electrical decoupling of the fluid temperature control unit from the power network is achieved, which has a positive effect on use in hyper/hypothermia systems since network feedback and leakage currents can be reduced. Attention must be paid in this respect to the fact that as medical-technical systems, hyper/hypothermia systems are subject to particularly critical specifications, which is why the decoupling of the fluid temperature control unit from the local power network that is achieved by the power supply unit according to the invention is to be regarded as positive.

Supplying the electrical consumers of the fluid temperature control unit with direct current offers the possibility of a more precise control during operation since a precise power control for each individual electrical consumer can take place, for example, with the aid of inverters. This is true not only for the heater/cooler of the fluid temperature control unit but also for the pumps which are generally electromotively driven. Overall, the clearly improved controllability of the temperature control device according to the invention leads to a noise-reducing scenario when used in a hyper/hypothermia treatment.

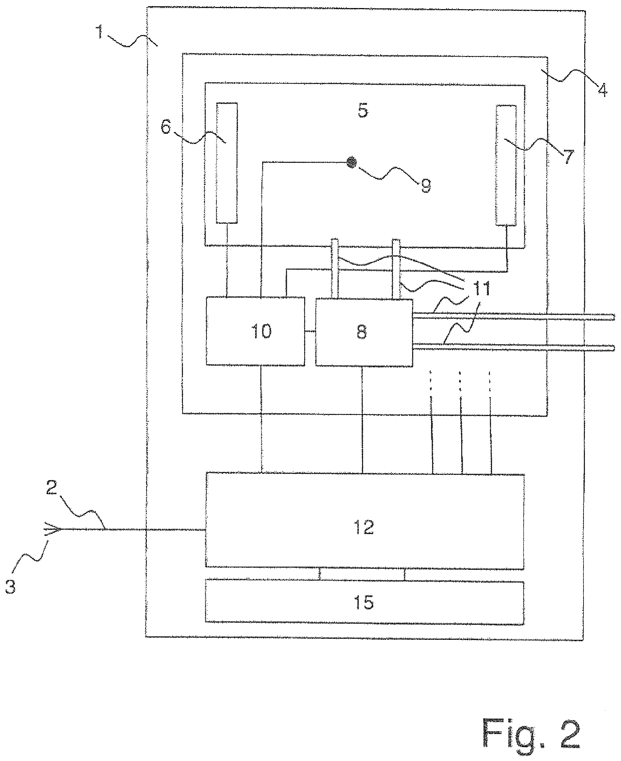

The further embodiment of a temperature control device 1 according to the invention which is shown in Fig. 2 additionally comprises a battery 15 for supplying the electrical consumers of the fluid temperature control unit 4 with power. The battery 15 is connected to the power supply unit 12 and is charged by this unit when the supply of power occurs via the power network. As a result, a fail-safe supply of direct current to the electrical consumers can be effected in the case that, for example, the local power network is subject to fluctuations for fails completely, since in this case the battery 15 can supply the power supply unit 12. Thus, designing the temperature control device with direct current consumers makes it possible to ensure the continuous operation of the temperature control device in a surgical environment.

## Claims

1. Temperature control device (1) for use in fluid-based hyper/hypothermia systems, comprising:
a connection unit (2) for connecting the device to a local power network (3); and
a fluid temperature control unit (4) for heating or cooling a fluid, said fluid temperature control unit (4) comprising:
- a fluid container (5),
- a heater (6) for heating said fluid in said fluid container (5)
- a cooler (7) for cooling said fluid in said fluid container (5)
- a supply pump (8) for removing said fluid from said fluid container (5) and for conveying it outside such that the fluid can be used in the hyper/hypothermia system,
- a temperature sensor (9) and
- a temperature controller (10); and
a power supply unit (12), via which all of the electrical consumers (5, 6, 7, 8, 10) of the fluid temperature control unit (4) are electrically supplied with power irrespective of the local power network (3), and which effects the supply of the consumers (6, 7, 8, 10) with direct current,
wherein the electrical consumers of the fluid temperature control unit (4) comprise said supply pump (8) with a direct current motor and said cooler (7) with a direct current compressor
and **characterized in that** the heater (6) is provided in the fluid container (5), the cooler (7) is provided in the fluid container (5) and the temperature sensor (9) is provided in the fluid container (5).

2. A temperature control device according to claim 1 **characterised in that** the power supply unit (12) is realised as a standard power supply.

3. A temperature control device according to claim 2, **characterised in that** the power supply unit (12) is realised as a switched-mode power supply.

4. A temperature control device according to one of claims 1 to 3, **characterised in that** the temperature control device (1) comprises a battery (15) for supplying the electrical consumers (6, 7, 8, 10) of the fluid temperature control unit (4), said battery being connected to the power supply unit (12) so as to be charged by said unit or to supply said unit.

## Patentansprüche

1. Temperatursteuervorrichtung (1) zur Verwendung in fluidbasierten Hyper-/Hypothermie-Systemen, mit:
einer Anschlusseinheit (2) für den Anschluss der Vorrichtung an ein örtliches Stromnetz (3); und
einer Fluidtemperatur-Steuereinheit (4) zum Erwärmen oder Abkühlen eines Fluids, wobei die Fluidtemperatur-Steuereinheit (4) umfasst:
- einen Fluidbehälter (5),
- einen Heizer (6) zum Erwärmen des Fluids in dem Fluidbehälter (5) und bereitgestellt in dem Fluidbehälter (5),
- einen Kühler (7) zum Abkühlen des Fluids in dem Fluidbehälter (5) und bereitgestellt in dem Fluidbehälter (5),
- eine Förderpumpe (8) zum Entfernen des Fluids aus dem Fluidbehälter (5) und zum nach außen Befördern, so dass das Fluid in dem Hyper-/Hypothermie-System verwendet werden kann,
- einen Temperatursensor (9) und bereitgestellt in dem Fluidbehälter (5), und
- eine Temperatursteuerung (10); und
eine Stromversorgungseinheit (12), über die alle elektrischen Verbraucher (5, 6, 7, 8, 10) der Fluidtemperatur-Steuereinheit (4) elektrisch mit Strom versorgt werden, unabhängig von dem örtlichen Stromnetz (3), und die die Versorgung der Verbraucher (6, 7, 8, 10) mit Gleichstrom bewerkstelligt,
wobei die elektrischen Verbraucher der Fluidtemperatur-Steuereinheit (4) die Förderpumpe (8) mit einem Gleichstrommotor und den Kühler (7) mit einem Gleichstromkompressor umfassen,
und **dadurch gekennzeichnet, dass** der Heizer (6) in dem Fluidbehälter (5) bereitgestellt ist, der Kühler (7) in dem Fluidbehälter (5) bereitgestellt ist und der Temperatursensor (9) in dem Fluidbehälter (5) bereitgestellt ist.

2. Temperatursteuervorrichtung nach Anspruch 1, **dadurch gekennzeichnet, dass** die Stromversorgungseinheit (12) als Standardnetzteil realisiert ist.

3. Temperatursteuervorrichtung nach Anspruch 2, **dadurch gekennzeichnet, dass** die Stromversorgungseinheit (12) als Schaltnetzteil realisiert ist.

4. Temperatursteuervorrichtung nach einem der Ansprüche 1 bis 3, **dadurch gekennzeichnet, dass** die Temperatursteuervorrichtung (1) eine Batterie (15) zur Versorgung der elektrischen Verbraucher (6, 7, 8, 10) der Fluidtemperatur-Steuereinheit (4) umfasst, wobei die Batterie mit der Stromversorgungseinheit (12) verbunden ist, um durch diese aufgeladen zu werden oder um diese zu speisen.

## Revendications

1. Dispositif de régulation de température (1) destiné à être utilisé dans des systèmes d'hyper/hypothermie à base de fluide, comprenant :
une unité de connexion (2) pour connecter le dispositif à un réseau électrique local (3) ; et
une unité de régulation de température de fluide (4) pour chauffer ou refroidir un fluide, ladite unité de régulation de température de fluide (4) comprenant :
- un conteneur de fluide (5),
- un élément chauffant (6) pour chauffer ledit fluide dans ledit conteneur de fluide (5),
- un refroidisseur (7) pour refroidir ledit fluide dans ledit conteneur de fluide (5),
- une pompe d'alimentation (8) pour retirer ledit fluide dudit conteneur de fluide (5) et le transporter à l'extérieur de telle sorte que le fluide puisse être utilisé dans le système d'hyper/hypothermie,
- un capteur de température (9), et
- un régulateur de température (10) ; et
une unité d'alimentation électrique (12), par l'intermédiaire de laquelle tous les consommateurs électriques (5, 6, 7, 8, 10) de l'unité de régulation de température de fluide (4) sont alimentés électriquement en énergie indépendamment du réseau électrique local (3), et qui assure l'alimentation des consommateurs (6, 7, 8, 10) en courant continu,
dans lequel les consommateurs électriques de l'unité de régulation de température de fluide (4) comprennent ladite pompe d'alimentation (8) avec un moteur à courant continu et ledit refroidisseur (7) avec un compresseur à courant continu,
et **caractérisé en ce que** l'élément chauffant (6) est fourni dans le conteneur de fluide (5), le refroidisseur (7) est fourni dans le conteneur de fluide (5), et le capteur de température (9) est fourni dans le conteneur de fluide (5).

2. Dispositif de régulation de température selon la revendication 1, **caractérisé en ce que** l'unité d'alimentation électrique (12) est réalisée comme une alimentation standard.

3. Dispositif de régulation de température selon la revendication 2, **caractérisé en ce que** l'unité d'alimentation électrique (12) est réalisée comme une alimentation en mode commuté.

4. Dispositif de régulation de température selon l'une des revendications 1 à 3, **caractérisé en ce que** le dispositif de régulation de température (1) comprend une batterie (15) pour alimenter les consommateurs électriques (6, 7, 8, 10) de l'unité de régulation de température de fluide (4), ladite batterie étant connectée à l'unité d'alimentation électrique (12) de manière à être chargée par ladite unité, ou à alimenter ladite unité.
